(19) Europäisches Patentamt
European Patent Office
Office européen des brevets

(11) Veröffentlichungsnummer : **0 503 347 B1**

(12) # EUROPÄISCHE PATENTSCHRIFT

(45) Veröffentlichungstag der Patentschrift : **23.03.94 Patentblatt 94/12**

(51) Int. Cl.$^5$ : **B01J 23/56,** B01J 23/64, C07C 209/72

(21) Anmeldenummer : **92103092.0**

(22) Anmeldetag : **24.02.92**

(54) **Verfahren zur Herstellung eines Gemisches von, gegebenenfalls substituiertem, Cyclohexylamin und gegebenenfalls substituiertem Dicyclohexylamin unter Ver- wendung eines Palladium/Al2O3 Katalysators.**

(30) Priorität : **08.03.91 DE 4107395**

(43) Veröffentlichungstag der Anmeldung : **16.09.92 Patentblatt 92/38**

(45) Bekanntmachung des Hinweises auf die Patenterteilung : **23.03.94 Patentblatt 94/12**

(84) Benannte Vertragsstaaten : **BE CH DE FR GB IT LI**

(56) Entgegenhaltungen :
**EP-A- 0 100 267**
**EP-A- 0 126 676**
**EP-A- 0 324 983**
**EP-A- 0 324 984**
**EP-A- 0 351 661**
**DE-A- 3 809 226**

(56) Entgegenhaltungen :
**US-A- 3 983 072**
**US-A- 4 152 351**
**PATENT ABSTRACTS OF JAPAN, Band 11, Nr. 192 (C-429)[2639], 19. Juni 1987; & JP-A-62-14 944 (NIKKI UNIVERSAL CO.) 23-01-1987**

(73) Patentinhaber : **BAYER AG**
**D-51368 Leverkusen (DE)**

(72) Erfinder : **Immel, Otto, Dr.**
**Immenhof 26**
**W-4150 Krefeld (DE)**
Erfinder : **Darsow, Gerhard, Dr.**
**In den Tannen 39**
**W-4150 Krefeld (DE)**
Erfinder : **Waldmann, Helmut, Dr.**
**Henry-Th.von-Böttinger-Strasse 15**
**W-5090 Leverkusen 1 (DE)**
Erfinder : **Petruck, Gerd-Michael, Dr.**
**Lechstrasse 6**
**W-4006 Erkrath 2 (DE)**

**EP 0 503 347 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft Verfahren zur Herstellung eines Gemisches von, gegebenenfalls substituiertem, Cyclohexylamin und, gegebenenfalls substituiertem, Dicyclohexylamin durch katalytische Hydrierung von, gegebenenfalls substituiertem, Anilin unter Einsatz eines Palladium-Trägerkatalysators.

Es ist bekannt, Cyclohexylamin durch Druckhydrierung von Anilin herzustellen. Für diese Hydrierung werden Kobalt-Katalysatoren, die einen basischen Zusatz enthalten (GB 969 542), sowie Raney-Kobalt (JP 68/03180) eingesetzt. Nach US 3.636.108 wird zur Kernhydrierung aromatischer Aminoverbindungen ein mit Alkali moderierter Ruthenium-Katalysator auf einem inerten Trägermaterial verwendet, wobei man zusätzlich $NH_3$ und gegebenenfalls ein Lösungsmittel einsetzt. Ein weiteres Verfahren zur Druckhydrierung von Anilin zu Cyclohexylamin ist in DE-AS 11 06 319 beschrieben, bei dem ebenfalls ein Ruthenium-Katalysator verwendet wird. In diesem Verfahren wird mitentstehendes Dicyclohexylamin dem Einsatzmaterial wieder zugesetzt; das Verfahren bringt beträchtliche Verluste durch die gleichzeitige Entstehung von Cyclohexan. EP 53 818 hält schließlich Palladium-Trägerkatalysatoren zur Druckhydrierung von Anilin für günstiger als Ruthenium-Katalysatoren; die dort beschriebenen Katalysatoren enthalten Zusätze, die entweder einer Gruppe von basischen Verbindungen der Alkalimetalle, Erdalkalimetalle und Seltenerdmetalle entstammen oder einer anderen Gruppe, die die Metalle Fe, Ni, Co, Mn, Zn, Cd und Ag umfaßt. Diese Katalysatoren erlauben die Reduktion von substituierten Anilinen zu den zugehörigen Cyclohexylaminen; die zugehörigen Dicyclohexylamine fehlen jedoch völlig.

Bei allen beschriebenen Druckhydrierungsverfahren von Anilin entsteht das Dicyclohexylamin neben dem Cyclohexylamin lediglich als Nebenprodukt oder überhaupt nicht. Um Dicyclohexylamin in größeren Mengen zu erhalten, wird es nach separaten Verfahren hergestellt. So kann es beispielsweise durch Druckhydrierung von Diphenylamin unter Verwendung eines Ruthenium-$Al_2O_3$-Katalysators gewonnen werden (DE-AS 11 06 319). Weiterhin entsteht Dicyclohexylamin bei der Umsetzung von Cyclohexanon mit Cyclohexylamin in Gegenwart von Palladium auf Kohle bei einem Wasserstoffdruck von 4 bar (FR 1.333.692). In einem umständlichen Verfahren kann Dicyclohexylamin aus dem Hydrierprodukt des Anilins an einem Nickelkatalysator durch fraktioniertes Auskondensieren gewonnen werden. Aus dem zurückbleibenden Gemisch wird ein Teil des mitentstandenen Ammoniaks entfernt und der Rest wieder in die Reaktion zurückgeführt (DE-PS 805 518).

Ein gemeinsames Problem aller Verfahren zur Kernhydrierung aromatischer Amine besteht in der zum Teil beträchtlichen Bildung von Cyclohexan als nicht weiter verwendbarem Nebenprodukt.

In neueren Verfahren, z.B. dem nach EP-A 324 983 wird ein Pd/Ru-Katalysator eingesetzt, der verbesserte, aber noch nicht ausreichende Mengen an Dicyclohexylamin ergibt. Der eingesetzte $Al_2O_3$-Träger ist mit einer Cr/Mn-Oxidschicht bedeckt, zu deren Herstellung giftige Cr-Verbindungen erforderlich sind.

Nach EP-A 324 984 verwendet man für die Hydrierung von Anilin Ru/Pd auf einem $Al_2O_3$-Träger, der basische Alkalimetallverbindungen enthält. Hierbei entsteht zwar Dicyclohexylamin als Hauptprodukt, aber die Umsetzung verläuft mit geringer Raum-Zeit-Ausbeute.

Für die gleiche Hydrieraufgabe wird nach EP-A 351 661 auch Ru allein verwendet, das sich auf einem Ce/Mn/$Al_2O_3$-Träger befindet. Mit Ru allein entstehen jedoch keine ausreichenden Mengen an Dicyclohexylamin; hingegen setzt bei erhöhten Temperaturen eine exotherme hydrogenolytische Spaltung der Reaktionsprodukte ein, die zu einer gefährlichen Wärmeentwicklung und weiterer Beschleunigung der Spaltreaktionen führen.

Es bestand daher der Wunsch, ein neues, auch in technischem Maßstab brauchbares Verfahren zu entwickeln, nach welchem in einer Reaktionsstufe sowohl Cyclohexylamin als auch Dicyclohexylamin in einem gewünschten Mengenverhältnis hergestellt werden können, in welchem der Verlust durch die unerwünschte Bildung von Cyclohexan zurückgedrängt wird und in welchem weiterhin die Standzeit des verwendeten Katalysators verbessert ist.

Überraschenderweise wurde nun gefunden, daß die genannten Anforderungen durch den Einsatz des im folgenden gekennzeichneten Palladium-Trägerkatalysators erfüllt werden, der einen $Al_2O_3$-Träger besitzt, der eine Kombination von Verbindungen der Seltenen Erdmetalle (III. Nebengruppe des Periodensystems der Elemente) und des Mangans enthält.

Die Erfindung betrifft demnach die Verwendung eines Palladium-Katalysators auf einem $Al_2O_3$-Träger, der dadurch gekennzeichnet ist, daß ein $\alpha$- oder $\gamma$-$Al_2O_3$ zunächst mit mindestens einer Verbindung von Seltenen Erdmetallen (III. Nebengruppe des Periodensystems der Elemente) und mit mindestens einer Verbindung des Mangans behandelt wird, wobei die Menge der Seltenen Erdmetalle und des Mangans insgesamt 0,05 bis 8 Gew.-%, bevorzugt 0,2 bis 5 Gew.-%, bezogen auf das Gesamtgewicht des Katalysators, und das Gewichtsverhältnis von Seltenen Erdmetallen und Mangan 5:1 bis 1:5, bevorzugt 10:9 bis 1:2, betragen und danach mit einer solchen Menge mindestens einer Palladiumverbindung behandelt wird, daß der Palladiumgehalt 0,05-5 Gew.-%, bevorzugt 0,05-4 Gew.-%, besonders bevorzugt 0,1-3 Gew.-%, bezogen auf das Gesamtgewicht

des Katalysators, beträgt.

Der erfindungsgemäß eingesetzte Katalysator enthält demnach als Träger ein $Al_2O_3$, welches mit Verbindungen von Seltenen Erdmetallen (III. Nebengruppe des Periodensystems der Elemente) und des Mangans behandelt worden ist. Als $Al_2O_3$ kommen die $\alpha$- und die $\gamma$-Modifikation, besonders bevorzugt die $\gamma$-Modifikation, in Frage. Der Träger weist einen Gehalt an Seltenem Erdmetall und Mangan von zusammen 0,05-8 Gew.-%, bevorzugt 0,2-5 Gew.-%, bezogen auf das Gesamtgewicht des Katalysators auf. Das Gewichtsverhältnis von Seltenem Erdmetall zu Mangan beträgt 5:1-1:5, bevorzugt 10:9-1:2. Als Seltene Erdmetalle werden die Elemente der III. Nebengruppe des Periodensystems, wie Scandium, Yttrium, Lanthan und die Lanthaniden verstanden. In bevorzugter Weise werden Yttrium, Lanthan, Cer, Praseodym, Neodym und Dysprosium, in besonders bevorzugter Weise Cer und Lanthan und in ganz besonders bevorzugter Weise Cer verstanden. Das Cer kann hierbei mit anderen Lanthaniden, beispielsweise mit Lanthan, Praseodym, Neodym, Dysprosium, oder mit Yttrium vergesellschaft sein. Eine solche Vergesellschaftung ist dem Fachmann im übrigen für alle genannten Seltenen Erdmetalle geläufig.

Zur Herstellung des erfindungsgemäß eingesetzten Katalysators kann so vorgegangen werden, daß auf ein $\alpha$- oder $\gamma$-$Al_2O_3$ in Form von Strangpreßlingen, Pillen oder Kugeln mit Abmessungen von etwa 2-10 mm Verbindungen der Seltenen Erdmetalle und des Mangans aufgebracht werden, der so beaufschlagte Träger nach dem Trocknen auf 200-450°C erhitzt wird und anschließend mit einer Lösung eines Palladiumsalzes getränkt oder besprüht wird, wonach sich eine erneute Trocknungsphase anschließt.

Das Aufbringen von Verbindungen der Seltenen Erdmetalle und des Mangans auf den Katalysatorträger kann beispielsweise durch bloßes Tränken oder Sprühen mit geeigneten Salzen der Seltenen Erdmetalle und des Mangans erfolgen, woran sich eine Trocknungsphase und die genannte Erhitzungsphase bei 200-450°C anschließen. Hierbei werden die Salze der Seltenen Erdmetalle und des Mangans in fest auf dem Katalysatorträger haftende Verbindungen übergeführt, ohne daß eine Spinellbildung eintritt. Das Aufbringen von Verbindungen der Seltenen Erdmetalle und des Mangans kann jedoch auch durch gemeinsames Ausfällen eines Seltenerd- Mangan-Hydroxidgemisches aus Seltenerd- und Mangansalzen auf dem Träger mit Alkalilauge oder Ammoniak und gegebenenfalls anschließendes Auswaschen der löslichen Anteile mit Wasser erfolgen. Als Seltenerd- und Mangansalze kommen insbesondere die Sulfate, Chloride, Acetate und/oder Nitrate der genannten Elemente in Betracht.

Nach dem Aufbringen der Seltenerd- und Manganverbindungen und gegebenenfalls nach der beschriebenen Ausfällung (und der damit verbundenen Auswaschung wasserlöslicher Verbindungen) wird der so behandelte Träger zunächst getrocknet, bevor er auf höhere Temperaturen (etwa 200-450°C, bevorzugt 250-430°C) erhitzt wird. Dieses Erhitzen erfolgt in einer Zeit von 1-120 Stunden. Während dieser Zeit kann die Temperatur im angegebenen Bereich von niederen auf höhere Werte erhöht werden.

Nach der beschriebenen Temperung wird der mit Verbindungen der Seltenen Erdmetalle und des Mangans beaufschlagte Katalysatorträger mit einer Palladium enthaltenden Lösung getränkt. Hierbei kann so vorgegangen werden, daß das Palladium, beispielsweise in Form wäßriger Lösungen des Chlorids, Nitrats, Acetats oder eines anderen geeigneten Salzes auf den Träger aufgetränkt oder aufgesprüht wird, gefolgt von einer Trocknung. Gegebenenfalls können die Palladiumsalze, wie beispielsweise Pd-Acetat, auch in organischen Lösungsmitteln wie Methanol, Methylenchlorid, Acetonitril oder Dioxan in Lösung gebracht und so aufgetränkt werden. Man kann jedoch auch vor der Trocknung den mit Palladiumsalzen getränkten Träger mit einer Lösung der obengenannten basischen Verbindung behandeln, wobei das Palladium als Oxid oder Hydroxid ausfällt. Auch hier schließt sich eine Trocknung an. Danach steht der Katalysator grundsätzlich zum Einsatz zur Verfügung. In bevorzugter Weise wird er jedoch vor seinem Einsatz, besonders bevorzugt nach Anordnung im Hydrierreaktor, durch Behandlung mit Wasserstoff bei einer Temperatur von 150-350°C aktiviert. Nach oder vor der Aktivierung kann es wünschenswert sein, Anionen wie Chlorid, Nitrat, Acetat oder andere und gegebenenfalls die Kationen der zur Ausfällung benutzten basischen Verbindungen durch eine Wasserwäsche zu entfernen.

Man kann jedoch auch den mit Verbindungen der Seltenen Erdmetalle und des Mangans beaufschlagten Katalysatorträger zunächst mit der Lösung einer der genannten basischen Verbindungen tränken, anschließend trocknen und auf den so vorbehandelten, basisch eingestellten Katalysatorträger Lösungen von Palladiumsalzen auftragen, wobei im Moment der Tränkung auch die Ausfällung des Palladiums in Form seines Oxids oder Hydroxids erfolgt. Auch hierbei ist nach einer abschließenden Trocknung der Katalysator grundsätzlich einsatzbereit, kann jedoch bevorzugt in der beschriebenen Weise vorab mit Wasserstoff bei der genannten Temperatur aktiviert werden.

Ein zur Fällung des Palladiums als Oxid oder Hydroxid mit basischen Verbindungen beaufschlagter Katalysator ist grundsätzlich auch in Gegenwart der Reste solcher alkalischer Verbindungen betriebsbereit. In bevorzugter Weise wird jedoch die beschriebene Wasserwäsche vorgenommen.

Die Tränkung oder das Besprühen des $Al_2O_3$-Trägers mit den genannten Stoffen und die hierfür erforderlichen Arbeitsgeräte sind dem Fachmann bekannt; ebenso bekannt ist die Einstellung der gewünschten Beaufschlagung durch die Wahl der Menge und Konzentration der Lösungen der genannten Elemente.

Die erfindungsgemäß eingesetzten Katalysatoren können in hervorragender Weise zur Kernhydrierung von gegebenenfalls substituierten Anilinen unter erhöhtem Druck eingesetzt werden, wobei in besonders überraschender Weise die gezielte Herstellung von Dicyclohexylamin in größeren Mengen neben gleichzeitig entstehendem Cyclohexylamin erfolgt. Die erfindungsgemäß eingesetzten Katalysatoren zeigen gegenüber einem nicht mit Verbindungen der Seltenen Erdmetalle und des Mangans hergestellten reinen Palladium-Trägerkatalysator die für kontinuierliche technische Prozesse erforderliche hohe Standzeit.

Damit ist erfindungsgemäß ein Verfahren zur Herstellung eines Gemisches von gegebenenfalls substituiertem Cyclohexylamin und gegebenenfalls substituiertem Dicyclohexylamin durch Hydrierung von gegebenenfalls substituiertem Anilin mit Wasserstoff in Gegenwart des oben beschriebenen Katalysators möglich, bei welchem man im Bereich von 150-300°C, bevorzugt 180-280°C, besonders bevorzugt 150-240°C bei einem Druck von 50-500 bar, bevorzugt 100-400 bar, besonders bevorzugt 150-350 bar, arbeitet.

Die exotherm verlaufende Hydrierung kann auf einem relativ hohen Temperaturniveau erfolgen, was für eine technische Energierückgewinnung von großer Bedeutung ist.

Die Hydrierung an den erfindungsgemäß eingesetzten Katalysatoren kann diskontinuierlich oder kontinuierlich, für technische Zwecke in bevorzugter Weise kontinuierlich vorgenommen werden; hierbei arbeitet man mit einer fest angeordneten Katalysatorschüttung in der Rieselphase.

Als Katalysatorbelastung wird eine Menge von 0,05-2 kg, bevorzugt 0,1-1 kg, besonders bevorzugt 0,15-0,8 kg Anilin pro Liter Katalysator pro Stunde eingestellt. Eine geringe Veränderung des erzielten Anilinumsatzes durch veränderte Aktivität des Katalysators im Laufe längerer Reaktionsperioden kann durch ein geringes Nachstellen der Reaktionstemperatur oder der anderen Parameter ausgeglichen werden. Diese Verhältnisse können anhand der Analytik des Reaktionsgemisches verfolgt werden.

Als Einsatzmaterialien kommen im Sinne der folgenden Reaktionsgleichung Anilin und substituierte Aniline in Betracht, die zu den korrespondierenden Cyclohexylaminen und Dicyclohexylaminen umgesetzt werden:

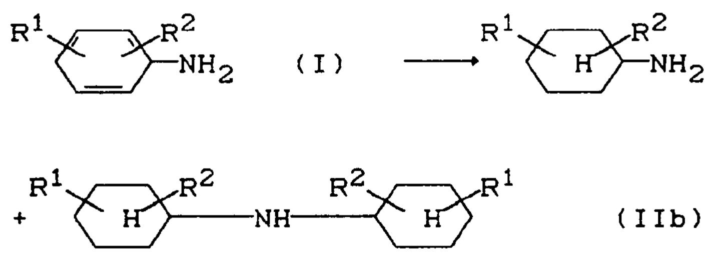

(I) (IIa)

(IIb)

Die Reste $R^1$ und $R^2$ haben unabhängig voneinander die Bedeutung von Wasserstoff, $C_1$-$C_4$-Alkyl oder $C_1$-$C_4$-Alkoxy. Beispiele für die genannten Alkyl- bzw. Alkoxysubstituenten sind: Methyl, Ethyl, Propyl, Isopropyl, Butyl, Isobutyl, Methoxy, Ethoxy, Propoxy, Isopropoxy, Butoxy oder Isobutoxy. In bevorzugter Weise haben die genannten Substituenten 1-2 C-Atome, besonders bevorzugt handelt es sich um Methyl bzw. Methoxy. In weiterhin bevorzugter Weise hat einer der Substituenten $R^1$ und $R^2$ die Bedeutung Wasserstoff, während der andere Substituent Alkyl bzw. Alkoxy im genannten Umfang bedeutet.

In besonders bevorzugter Weise richtet sich das Verfahren auf die Kernhydrierung von nicht substituiertem Anilin.

Cyclohexylamine und Dicyclohexylamine des genannten Bedeutungsumfanges finden Verwendung zur Herstellung von Alterungsschutzmitteln für Kautschuke und Kunststoffe, als Korrosionsschutzmittel, sowie als Vorprodukte für Pflanzenschutzmittel und Textilhilfsmittel.

Beispiel 1

200 g eines handelsüblichen $\gamma$-$Al_2O_3$ mit einer spezifischen Oberfläche von 350 $m^2/g$ und einem Kugeldurchmesser von 2 bis 6 mm wurden mit einer Lösung getränkt, die aus
12,4 g $Ce(NO_3)_3 \cdot 6\ H_2O$,
18,28 g $Mn(NO_3)_2 \cdot 4\ H_2O$ und
75 g Wasser
hergestellt worden war. Das getränkte $Al_2O_3$ wurde im Wasserstrahlvakuum 18 Stunden bei 120°C getrocknet und anschließend 3 Stunden lang bei 400°C getempert.

100 g des so hergestellten Katalysatorträgers wurden mit einer Lösung getränkt, die aus 4,16 g Pd-Acetat

und 30 g Dioxan hergestellt worden war. Der Katalysator wurde 18 Stunden bei 100°C getrocknet und anschließend 3 Stunden bei 300°C im $H_2$-Strom aktiviert. Bei der Tränkung wurden 2 Gew.-% Pd, bezogen auf das Gesamtgewicht des Katalysators, aufgebracht. Zur Hydrierung von Anilin wurden 60 ml (48,8 g) des Pd-Katalysators in ein senkrecht angeordnetes Druckrohr (Durchmesser 14 mm, Länge 70 cm) gebracht, das mit einem Ölthermostat beheizt wurde. Zur weiteren Aktivierung des Katalysators wurde er 3 Stunden bei 300°C und 270 bar Wasserstoff behandelt. Dabei wurden 100 l Wasserstoff/h entspannt.

Danach wurde die Temperatur auf etwa 195°C gesenkt und bei 280 bar Anilin und Wasserstoff von oben auf den Katalysator geleitet. Die Flüssigkeit rieselte über den Katalysator nach unten in einen Abscheider. Am Kopf des Abscheiders wurden 90 bis 100 l/h Wasserstoff entspannt.

Der Anilindurchsatz entsprach einer Katalysatorbelastung von 0,24 bis 0,33 g Anilin/ml Kat. x h und wurde in diesem Bereich gehalten.

Das Hydrierprodukt wurde in regelmäßigen Zeitabständen aus dem Abscheider entnommen und analysiert. Dabei ergab sich folgende Produktzusammensetzung in Abhängigkeit von der Laufzeit und der Reaktionstemperatur bei einer Versuchsdauer von mehr als 1600 Stunden.

| Laufzeit (h) | Temp. (°C) | Anilin (%) | DHA* (%) | CHA* (%) | Nebenprodukte (%) |
|---|---|---|---|---|---|
| 65 | 196 | 0,5 | 84,2 | 15,1 | 0,2 |
| 186 | 194 | 1,2 | 86,4 | 12,0 | 0,4 |
| 474 | 191 | 1,0 | 86,1 | 12,6 | 0,3 |
| 667 | 203 | 0,2 | 84,2 | 15,5 | 0,1 |
| 811 | 203 | 0,1 | 84,2 | 15,6 | 0,1 |
| 909 | 202 | 0,2 | 84,3 | 15,4 | 0,1 |
| 1220 | 204 | 0,2 | 83,7 | 16,0 | 0,1 |
| 1621 | 201 | 0,1 | 83,3 | 16,4 | 0,2 |

*) DHA = Dicyclohexylamin; CHA = Cyclohexylamin

<u>Beispiel 2</u>

100 g des gemäß Beispiel 1 hergestellten Katalysatorträgers wurden mit einer Lösung getränkt, die aus 2,08 g Pd-Acetat und 30 g Dioxan hergestellt worden war. Der mit Pd (1 %) getränkte Katalysator wurde 18 Stunden bei 100°C getrocknet.

40 ml (34,5 g) des so hergestellten Katalysators wurden für die kontinuierliche Anilinhydrierung in ein Druckrohr gefüllt, und es wurde nach der in Beispiel 1 beschriebenen Weise verfahren. Zunächst wurde der Katalysator wieder bei 300°C und 270 bar aktiviert, bevor mit der kontinuierlichen Hydrierung von Anilin begonnen wurde.

Der Anilindurchsatz entsprach einer Katalysatorbelastung von 0,25 bis 0,41 g Anilin/ml·Kat. x h. Aus dem Druckabscheider wurden stündlich 90 bis 100 l Wasserstoff entspannt. Das Reaktionsprodukt zeigte in Abhängigkeit von der Hydriertemperatur und der Versuchsdauer folgende Zusammensetzung:

| Lauf-zeit (h) | Temp. (°C) | Anilin (%) | DHA* (%) | CHA* (%) | Neben-produkte (%) |
|---|---|---|---|---|---|
| 119 | 205 | 1,1 | 83,4 | 14,8 | 0,7 |
| 286 | 226 | 0,1 | 72,2 | 25,9 | 0,8 |
| 601 | 232 | - | 73,6 | 26,1 | 0,3 |
| 1098 | 231 | 0,9 | 74,2 | 24,6 | 0,3 |
| 1505 | 231 | 1,8 | 76,3 | 21,5 | 0,4 |
| 1892 | 244 | 0,2 | 69,1 | 30,6 | 0,1 |
| 2134 | 239 | 0,6 | 73,1 | 26,2 | 0,1 |

*) DHA = Dicyclohexylamin: CHA = Cyclohexylamin

Die Zusammensetzung des Reaktionsproduktes in Abhängigkeit von der Hydriertemperatur zeigt, daß selbst bei hohen Temperaturen um 240°C keine Verluste durch Hydrogenolyse auftreten.

## Patentansprüche

1. Verfahren zur Herstellung eines Gemisches von, gegebenenfalls substituiertem, Cyclohexylamin und, gegebenenfalls substituiertem, Dicyclohexylamin durch Hydrierung von, gegebenenfalls substituiertem, Anilin mit Wasserstoff in Gegenwart eines Palladium/$Al_2O_3$-Trägerkatalysators, dadurch gekennzeichnet, daß man einen Katalysator einsetzt, der dadurch gekennzeichnet ist, daß ein α- oder γ-$Al_2O_3$ zunächst mit mindestens einer Verbindung von seltenen Erdmetallen (III. Nebengruppe des Periodensystems der Elemente) und mit mindestens einer Verbindung des Mangans behandelt wird, wobei die Menge der Seltenen Erdmetalle und des Mangans insgesamt 0,05 bis 8 Gew.-%, bevorzugt 0,2 bis 5 Gew.-%, bezogen auf das Gesamtgewicht des Katalysators, und das Gewichtsverhältnis von Seltenen Erdmetallen und Mangan 5:1 bis 1:5, bevorzugt 10:9 bis 1:2, betragen und danach mit einer solchen Menge mindestens einer Palladiumverbindung behandelt wird, daß der Pd-Gehalt 0,05 bis 5 Gew.-%, bevorzugt 0,05 bis 4 Gew.-%, besonders bevorzugt 0,1 bis 3 Gew.-%, bezogen auf das Gesamtgewicht des Katalysators, beträgt und bei 150 bis 300°C und 50 bis 500 bar arbeitet.

2. Verfahren nach Anspruch 1, dadurch gekennzeichnet, daß als Seltenes Erdmetall eines oder mehrere aus der Gruppe Yttrium, Lanthan, Cer, Praseodym, Neodym und Dysprosium, bevorzugt aus der Gruppe Cer und Lanthan, eingesetzt wird (werden) und besonders bevorzugt Cer eingesetzt wird.

3. Verfahren nach Anspruch 1, dadurch gekennzeichnet, daß man eine Katalysatorbelastung von 0,05 bis 2 kg, bevorzugt 0,1 bis 1 kg, besonders bevorzugt 0,15 bis 0,8 kg Anilin pro Liter Katalysator pro Stunde einstellt.

4. Verfahren nach Anspruch 1, dadurch gekennzeichnet, daß man ein Anilin der Formel

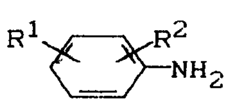

einsetzt, in der

$R^1$ und $R^2$ unabhängig voneinander Wasserstoff, $C_1$-$C_4$-Alkyl oder $C_1$-$C_4$-Alkoxy bedeuten.

## Claims

1. Process for preparing a mixture of optionally substituted cyclohexylamine and optionally substituted dicyclohexylamine by hydrogenation of optionally substituted aniline with hydrogen in the presence of a palladium/$Al_2O_3$-support catalyst, characterized in that the catalyst used is one characterized in that an α- or γ-$Al_2O_3$ is first treated with at least one compound of rare-earth metals (subgroup III of the Periodic Table of the Elements) and with at least one compound of manganese, the amount of the rare-earth metals and the manganese being in total from 0.05 to 8% by weight, preferably from 0.2 to 5% by weight, based on the total weight of the catalyst, and the weight ratio of rare-earth metals and manganese being from 5:1 to 1:5, preferably from 10:9 to 1:2, and is then treated with at least one palladium compound in an amount such that the Pd content is from 0.05 to 5% by weight, preferably from 0.05 to 4% by weight, particularly preferably from 0.1 to 3% by weight, based on the total weight of the catalyst, and the process is carried out at from 150 to 300°C and from 50 to 500 bar.

2. Process according to Claim 1, characterized in that the rare-earth metal(s) used is (are) one or more from the group comprising yttrium, lanthanum, cerium, praseodymium, neodymium and dysprosium, preferably from the group comprising cerium and lanthanum, and particularly preferably is cerium.

3. Process according to Claim 1, characterized in that a weight hourly space velocity of from 0.05 to 2 kg, preferably from 0.1 to 1 kg, particularly preferably from 0.15 to 0.8 kg, of aniline per litre of catalyst per hour is employed.

4. Process according to Claim 1, characterized in that an aniline of the formula

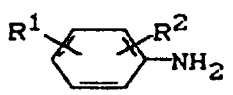

is used, in which

$R^1$ and $R^2$ independently of one another are hydrogen, $C_1$-$C_4$-alkyl or $C_1$-$C_4$-alkoxy.

## Revendications

1. Procédé de production d'un mélange de cyclohexylamine éventuellement substituée et de dicyclohexylamine éventuellement substituée par hydrogénation d'aniline éventuellement substituée, avec de l'hydrogène en présence d'un catalyseur au palladium fixé sur un support de $Al_2O_3$, caractérisé en ce qu'on utilise un catalyseur qui est caractérisé en ce qu'une alumine α ou γ est traitée tout d'abord avec au moins un composé de métaux des terres rares (sous-groupe III du Système Périodique de Classification des Eléments) et avec au moins un composé de manganèse, la quantité de métaux des terres rares et de manganèse s'élevant au total à 0,05 - 8 % en poids, de préférence à 0,2 - 5 % en poids, par rapport au poids total de catalyseur, et le rapport des poids de métaux des terres rares et de manganèse s'élevant à 5:1 à 1:5, de préférence à 10:9 - 1:2, puis traitée avec une quantité telle d'au moins un composé de palladium que la teneur en palladium s'élève à 0,05 - 5 % en poids, de préférence à 0,05 - 4 % en poids, notamment à 0,1 - 3 % en poids, par rapport au poids total de catalyseur, et on opère à une température de 150 à 300°C et à une pression de 50 à 500 bars.

2. Procédé suivant la revendication 1, caractérisé en ce qu'on utilise comme métal des terres rares un ou plusieurs métaux du groupe formé de l'yttrium, du lanthane, du cérium, du praséodyme, du néodyme et du dysprosium, de préférence du groupe formé du cérium et du lanthane, et on utilise très préférentiellement le cérium.

3. Procédé suivant la revendication 1, caractérisé en ce qu'on établit une charge de catalyseur de 0,05 à 2

kg, mieux encore de 0,1 à 1 kg, notamment de 0,15 à 0,8 kg d'aniline par litre de catalyseur par heure.

4. Procédé suivant la revendication 1, caractérisé en ce qu'on utilise une aniline de formule

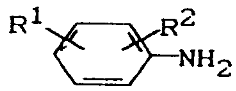

dans laquelle

$R^1$ et $R^2$ représentent, indépendamment l'un de l'autre, l'hydrogène, un groupe alkyle en $C_1$ à $C_4$ ou un groupe alkoxy en $C_1$ à $C_4$.